# EUROPEAN PATENT SPECIFICATION

(11) **EP 0 440 702 B1**
(45) Date of publication and mention of the grant of the patent: **13.04.1994**
(21) Application number: 89911914.3
(22) Date of filing: 27.10.1989
(51) Int. Cl.: A61K 9/18, A61K 9/14, A61K 47/20

(54) **A PHARMACEUTICAL COMPOSITION FOR RAPID RELEASE OF THE ACTIVE COMPONENT COMPRISING A SOLID DISPERSION AND A SURFACTANT**
ARZNEIMITTELZUSAMMENSETZUNG FÜR DIE SCHNELLE VERABREICHUNG DES AKTIVEN BESTANDTEILS, DER AUS EINER FESTEN DISPERSION UND EINEM OBERFLÄCHENAKTIVEN MITTEL BESTEHT
COMPOSITION PHARMACEUTIQUE A LIBERATION RAPIDE DU COMPOSANT ACTIF COMPRENANT UNE DISPERSION SOLIDE ET UN TENSIO-ACTIF

(30) Priority: 31.10.1988 SE 8803935
(43) Date of publication of application: 14.08.1991
(73) Proprietor: Porten Pharmaceutical AB, 756 51 Uppsala (SE)
(72) Inventor: NYSTRÖM, Christer, S-756 51 Uppsala (SE); SJÖKVIST, Eva, S-753 28 Uppsala (SE); WESTERBERG, Marie, S-172 34 Sundbyberg (SE)
(74) Representative: Hansson, Sven A.
(86) International application number: SE8900599
(87) International publication number: WO9004962

(56) References cited:
- EP-A- 0 012 523
- WO-A-87/00428
- US-A- 4 744 976
- Powder Technology, 1984, Iinoya et al., "Ordered Mixes of Hydrophobic Drugs and a directly Compressible Excipient-Dissolution and Uniformity Properties", pp. 734-741
- Pharmaceutisch Weekblad Scientific Edition, Vol. 6, 1984, E.J. de Jong and C.J. de Blaey, "A new approach of ordered mixing applied to digitoxin tablets", pp. 16-17

## Description

The present invention relates to a pharmaceutical composition which exhibits improved release of the pharmaceutical substance. More specifically, but not exclusively, the invention relates to a pharmaceutical composition which contains a considerable amount of active pharmaceutical substance but which, nevertheless, exhibits a high rate of pharmaceutical-substance release.

It is known to formulate pharmaceutical compositions as so-called ordered mixtures in which very fine particles of a hydrophobic pharmaceutical substance adhere to the surfaces of larger particles of a water-soluble carrier substance. The carrier particles dissolve in the presence of water and form a solution, wherewith the adherent particles of pharmaceutical substance disperse throughout the liquid. This eliminates the inherent tendency of the particles of hydrophobic pharmaceutical substance to collect into not-readily dissolvable and dispersible aggregates.

The so-called surface-area ratio, Rₛ,constitutes a measurement of the quantity of particulate therapeutically active agent which will adhere to the surfaces of the larger carrier particles. Rₛ is defined as the ratio of the projected surface of the adherent particles to the total external surface-area of the carrier particles. A more detailed description of how this surface-area ratio is determined is given by C. Nyström, J. Mazur and J. Sjögren in International Journal of Pharmaceutics, 10: pages 209-218 (1982).

It is found that with increasing values of the surface-area ratio Rₛ, the dissolution properties of the substance will be impaired to a commensurate degree and that the ordered mixture no longer provides an improvement. This is due to the fact that because of their hydrophobic properties, the particles of pharmaceutical substance prevent the penetration of water to the under-lying carrier particles, despite the fact that the adherent particles do not cover the whole of the surface of the carrier particles.

Endeavours have been made to improve the dissolution rate of such compositions by incorporating a pharmaceutical disintegrant or "explosive" substance of known kind. This has provided some improvement, since the carrier particles decompose more readily and since the formation of agglomerates from the particulate hydrophobic pharmaceutical substance is prevented. Good results are obtained at surface-area ratios of up to about 0.5, but at ratios above this value the dissolution rate again falls significantly. As in the case of compositions which lack the inclusion of a disintegrant, this fall in the dissolution rate is due to the fact that the fine particles of pharmaceutical substance form a dense network which cannot be penetrated by the water, because of the hydrophobic properties of the particles. The carrier particle with particles of pharmaceutical substance adhering thereto thus behaves as a large aggregate of solely pharmaceutical-substance particles.

It is evident that an improvement is highly desirable which will enable higher surface-area ratios to be achieved when mutually combining carrier particles and particles of pharmaceutical substance while maintaining good dissolution properties. Such an improvement would enable a pharmaceutical dosage unit, e.g. a tablet, to contain a large quantity of pharmaceutical substance without impairing its dissolution rate.

Another method of obtaining a composition comprising a finely-divided pharmaceutical substance in combination with a water-soluble carrier is to precipitate the pharmaceutical substance in a-fine particulate form from a solution of said substance in the carrier. For instance, the pharmaceutical substance can be soluble in the carrier at high temperature, but not-readily dissolved at room temperature, wherein a fine dispersion of the pharmaceutical substance is obtained in the carrier, by cooling the solution therein to room temperature under controlled conditions. This will result in a fine dispersion of the hydrophobic pharmaceutical substance in the water-soluble carrier in which the particles of pharmaceutical substance are mutually separated. Consequently, the formation of not-readily dissolved aggregates is prevented, when the composition is dissolved in water.

In this case, the carrier may consist of polyethylene glycol, which is able to dissolve many hydrophobic pharmaceutical substances in heat, these substances subsequently precipitating in a finely dispersed form when the carrier cools. The polyethylene glycol may be more or less highly viscous or preferably solid, at room temperature, depending on the degree of polymerisation.

However, it is found that the rate of dissolution decreases markedly when the proportion of pharmaceutical substance in such a dispersion exceeds a given value, so that the composition becomes less useful for the purpose intended. This is due to the fact that with increasing proportions of pharmaceutical substance, the hydrophobic particles in the dispersion come progressively closer together and ultimately form a water-impenetrable hydrophobic network. Thus a situation is reached which is analogous to the aforedescribed situation that occurs when particles of pharmaceutical substance are adhered to the surfaces of carrier particles.

Naturally, it is desirable to embody as much pharmaceutical substance as possible in dosage-unit form in order to obtain a good effect, even in the case of compositions in which the pharmaceutical composition is dispersed in a carrier. At the same time, it is also desirable to obtain a composition which will dissolve as quickly as possible, so as to achieve the effect intended quickly.

The present invention eliminates the aforesaid drawbacks to a large extent and provides a pharmaceutical composition which consists of finely-divided, particulate pharmaceutical substance in combination with a water-soluble carrier and which exhibits a rapid release rate of pharmaceutical substance even when the composition contains large quantities of said substance.

The invention thus relates to a pharmaceutical composition which enables rapid release of the pharmaceutical substance contained therein and which contains at least one finely-divided, hydrophobic pharmaceutical substance which is present in a finely dispersed or dissolved state in a water-soluble carrier together with a finely dispersed or dissolved surfactant, characterized in that said substance and said surfactant have seen incorporated into said carrier while the carrier has been heated to a molten state, to form an intimate mixture with each other, and said mixture has been cooled to solidify into said pharmaceutical composition., The surfactant is preferably present in an amount of 0.5 to 5 weight percent, and then preferably in an amount of 0.5 to 3 percent of the total weight of the composition.

In EP-A1-0 012 523 are disclosed pharmaceutical compositions comprising a mixture of a hydrophobic pharmaceutical agent and a water-soluble polymer. This mixture has then been treated with a minor amount of a wetting agent. This means that the wetting agent will only be present in the surface regions of the particles of the mixture, and will not be intimately mixed with the pharmaceutical agent, as is required according to the present invention.

In WO 87/00428 are disclosed pharmaceutical compositions of a benzodiazepin compound as an active agent, which is absorbed on a pharmaceutical carrier or excipient in fine particle form. To this composition may be added a surfactant. Only water-insoluble carriers are discussed.

Surfactants have been used previously to some extent in connection with pharmaceutical preparations. For instance, surfactants have often been added to the solvent, with the purpose of assisting dissolution of tablets. Because the surfactant lowers surface tension, the tablet is wetted more readily and the liquid will penetrate the tablet more easily, so as to increase the area of surface contact between liquid and solid phase. This increases the speed at which dissolution takes place. For example, it is found that an addition of 0.01 percent by weight Tween ^{R}₈₀ (polyethylene oxide (20) sorbitan monooleate) to an aqueous phase will provide the same surface-tension conditions as those conditions which are considered to exist in gastric juices.

Surfactants have also been used to promote the dissolution of pharmaceutical substances in micronized form. Such substances often form agglomerates, and consequently the surface-area available to the solvent becomes smaller, therewith resulting in a slower dissolution rate. The incorporation of a surfactant in the pharmaceutical preparation can result in improved wetting and therewith a faster dissolution rate.

Surfactants have also been used widely to promote the solubilization of not-readily dissolved lipophilic substances. When a surfactant is added to water in sufficient quantities, micelles are formed by the agglomeration of the surfactant molecules. The lipophilic hydrocarbon parts of the molecules in the micelles face inwards towards the interior of the micelles, whereas the hydrophilic parts face outwards towards the water phase. The interior of the micelles can therefore be considered to be an organic phase which is capable of acting as an organic solvent. Substances which are not-readily dissolved in water can then be dissolved in this organic phase and the "water solubility" of the not-readily dissolved substances can often be enhanced considerably through this solubilization.

The present invention employs a surfactant in a manner which is different in principle to the manner previously used. According to the invention, the surfactant is used together with a mixture of a water-soluble carrier and a finely-divided, hydrophobic pharmaceutical substance, and enables larger quantities of pharmaceutical substance to be combined with the carrier while still achieving rapid dissolution of the pharmaceutical substance.

The surfactant may be of an anionic, a cationic or a non-ionic type, although the anionic and non-ionic surfactants are preferred. In fact, the surfactant need only fullfil the requirement of being pharmaceutically acceptable and thus shall not cause undesirable side-effects in the part of the patient. Neither shall the surfactant be capable of reacting unfavourably with the pharmaceutical substance, carrier or other material present in the composition. Examples of suitable anionic surfactants are sulphuric-acid esters, such as sulphated monoglycerides and alkylsulphates, and then sodium lauryl sulphate in particular, substituted alkyl amides, such as sarcosinates, and hemi-esters, such as sulphur-succinates. Non-ionic surfactants may consist in polyalkoxy ethers, such as polyoxyethylene-polyoxypropylene block polymers, polyalkoxy esters, such as polyoxyethylene fatty acid esters and polyoxyethylene-oxide sorbitanic acid esters, and then Tween ^{R}₈₀ (polyoxy ethylene oxide sorbitan monoleate) for instance, and fatty acid esters of multi-valent alcohols, such as glyceryl esters and sorbitan esters. Other surfactants can be used, as will be understood by the person skilled in this field.

The active pharmaceutical substance or substances present in the composition will normally have a particle size of at most about 10 µm, and then preferably at most about 6 µm.

According to the invention, the hydrophobic pharmaceutical substance and the surfactant are present in a finely-dispersed form in the water-soluble carrier. The pharmaceutical substance and the carrier are normally so adapted to one another that the pharmaceutical substance will dissolve in the carrier at elevated temperatures but will not readily dissolve therein at lower temperatures, normally room temperature, such that the fine dispersion is obtained by precipitation of the pharmaceutical substance as the solution cools. The conditions which prevail during cooling of the solution can be adjusted in a manner to obtain a particle-size of appropriate fineness.

It is also conceivable for the pharmaceutical substance to be soluble in the water-soluble carrier at room temperature. In the case of the preferred embodiment, in which the carrier is solid at room temperature, there is obtained a solid solution of pharmaceutical substance in the carrier. This can be said to constitute the border case of a fine-dispersement of pharmaceutical substance in the carrier, and is also included in the invention.

According to the invention, a pharmaceutical composition shall also contain at least one surfactant in finely-dispersed form and in intimate mixture with the pharmaceutical substance. This can be achieved by mixing the surfactant together with the pharmaceutical substance in the liquid carrier at elevated temperature. In this case, the surfactant, similar to the pharmaceutical substance, is dissolved in the hot carrier and precipitates in a finely dispersed form as the solution cools, or the surfactant may, alternatively, be not-readily dissolvable in the carrier and is in a sufficiently finely-divided form at the stage of being mixed with the carrier. It is also conceivable for the surfactant to remain dissolved in the carrier, even at room temperature, without preventing the surfactant from exerting the desired effect when the composition is dissolved in water in use.

The carrier material used shall consist of a water-soluble material in which the pharmaceutical substance can be dispersed in a finely-divided state. In the preferred case, the pharmaceutical substance is soluble in the carrier at elevated temperature, but precipitates in a finely-divided form when the solution cools. It is preferred that the carrier is solid at room temperature although liquid at said temperature when the pharmaceutical substance is dissolved, since this facilitates handling of the pharmaceutical composition in its finished form.

It is also conceivable for the pharmaceutical substance to be not-readily soluble in the carrier at elevated temperature. In these cases, the finely-divided pharmaceutical substance is finely-dispersed mechanically in the liquid carrier at the elevated temperature, where-after the resultant mixture is allowed to cool while maintaining the dispersion until the carrier has solidified or has become sufficiently viscous for the dispersion to be maintained over an unlimited period of time.

The surfactant will also preferably exhibit similar dissolution and dispersion properties as the pharmaceutical substance or substances used, but need not perform in precisely the same manner as the substance or substances. The important criterion is that the final product includes an intimate mixture of pharmaceutical substance and surfactant in finely-divided form dispersed in the water-soluble carrier. Provided that this result is achieved, it is irrelevant if one or both of the components have been dissolved in the carrier or mechanically dispersed therein.

Several carrier materials which fullfil the requirements placed thereon are known to the person skilled in this art, and polyethylene glycol of varying molecular weights can be mentioned as an example of suitable carrier material. Polyethylene glycol having a molecular weight within the range of about 3000 to about 20000, and then particularly about 3000, has been found suitable because of its good dissolution properties in a molten state, while at the same time being solid and easily handled at room temperature. Examples of further carriers include xylitol and sorbitol.

The amount of pharmaceutical substance incorporated in the carrier can vary within wide limits, and is determined to a large extent by the solubility and dispersability of the substance in the carrier. It will be readily understood that the addition of surfactant in accordance with the invention affords the greatest advantages at high content values, at which the particles of the hydrophobic pharmaceutical substance would otherwise form a dense, hydrophobic network which prevents penetration of the water. The present invention affords practically no advantages at low contents, since the particles of pharmaceutical substance are then still spaced widely apart and are thus unable to prevent dissolution. For example, the pharmaceutical substance may be present in the carrier in an amount corresponding to from 5 to 50 percent by weight of the total pharmaceutical composition, and then preferably from 5 to 25 percent by weight, although these values are not critical.

The pharmaceutical compositions produced in accordance with the invention may be included in different types of pharmaceutical preparations. The preparations will preferably be intended for enteral administration, primarily for oral administration. The preparations are, for instance, in tablet, capsule, powder or granule form or in the form of suppositories for rectal administration. Pharmaceutical compositions prepared in accordance with the invention may also be used in preparations for external use, such as ointments or creams. Irrespective of the method of administration chosen, it is important that the preparation is essentially free from water, since the presence of water would result in premature dissolution of the pharmaceutical substance.

The pharmaceutical preparations can be formulated by combining the inventive pharmaceutical compositions with the conventional pharmaceutical additives and excipients normally used in the desired forms of the preparations, with the aid of known methods herefor. Such additions may comprise, for instance, additional carriers, preservatives, lubricants, glidants, dis-integrants, flavorants, dyestuffs and like substances, and are well known to the person skilled in this art.

The active pharmaceutical substances capable of being included in the inventive pharmaceutical compositions can vary very widely. Any pharmaceutical substance which, together with a surfactant, can be brought to a sufficiently small particle size and be caused to adhere to the surfaces of water-soluble carrier particles, or can be dispersed in a water-soluble carrier, can be used within the scope of the present invention. The advantages afforded by the present invention are obtained primarily when using hydrophobic pharmaceutical substances which are not-readily dissolved in water, although the degree of solubility in water can vary with the type of substance used, and the intention is not that the solubility of the active pharmaceutical substance or substances shall constitute a limitation of the invention. It is simple for one skilled in this art to establish by routine experiment whether or not a pharmaceutical substance can be used in a pharmaceutical composition according to the invention.

Benzodiazepines, ergotamine tartrate, isosorbide dinitrate and griseofulvin are examples of different types of active substance which can be used in conjunction with the present invention. This recitation of suitable active substances is not in any way exhaustive, however.

The invention will be illustrated more clearly with the aid of the following non-limitive working example and with reference to the accompanying drawings.

### Example

This example illustrates the use of a surfactant together with a finely-dispersed pharmaceutical substance in a water-soluble carrier.

10 percent by weight griseofulvin was dissolved in warm polyethylene glycole 3000 and the solution was divided into two batches. 1 percent by weight sodium lauryl sulphate was dissolved in one of the batches, whereafter the two batches were allowed to cool to room temperature. The griseofulvin precipitated in a finely-dispersed form having a particle size of about 3 µm.

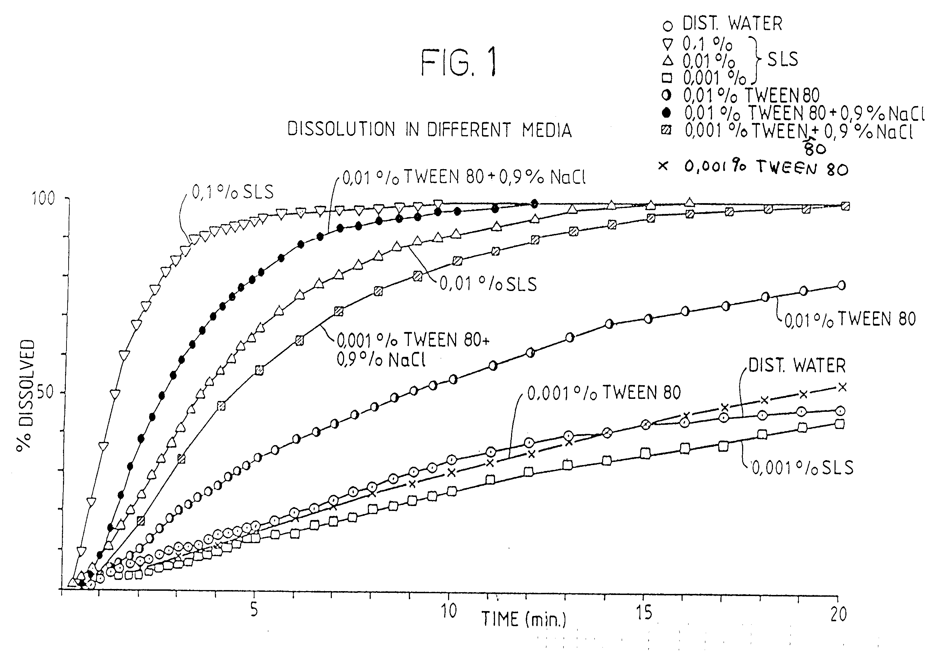

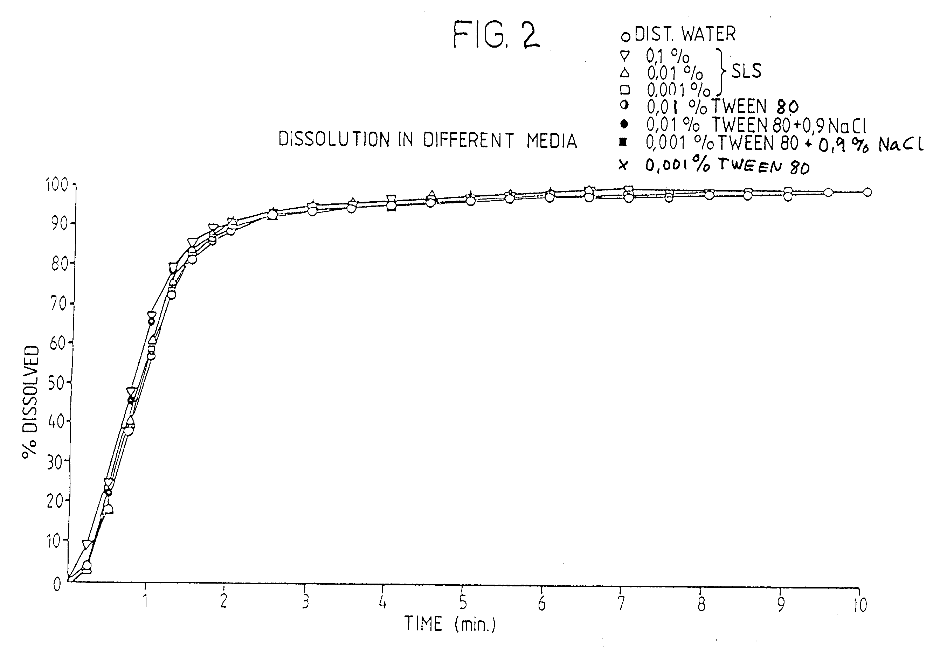

The dissolution rate of the two batches having a particle size of 300-500 µm was then determined in accordance with U.S.P. XXI, pages 1243-1244, the paddle method, in distilled water with various additives. These additives comprised sodium lauryl sulphate in quantities of 0.1, 0.01 and 0.001 percent by weight, Tween 80 ^{R} (polyethyleneoxy-disorbitane monooleate) in quantities of 0.01 and 0.001 percent by weight, and Tween 80 ^{R} in the same quantities but with a further addition of 0.9 percent by weight NaCl. The results of the dissolution tests carried out are set forth in Figures 1 and 2.

It will clearly be seen from Figure 1 that the surfactant content of the dissolution water is significant to the rate of dissolution when the composition itself contains no surfactant. Thus, at low surfactant contents of 0.001 percent by weight essentially the same rate of dissolution is obtained as that of pure water, and it is found that hardly half of the composition has dissolved after a lapse of 20 minutes. As expected, the dissolution rate increases with higher contents in the water solvent, although it will be noted that even when the amount of sodium lauryl sulphate present in the water is as high as 0.1 percent, it still takes about 10 minutes for all the composition to pass into solution. This is considered to be a good result when practising prior art techniques.

Figure 2 shows clearly that, quite unexpectedly, with an addition of 1 percent sodium lauryl sulphate in finely dispersed form in mixture with the pharmaceutical substance in the carrier, the dissolution rate is essentially independent of whether or not the water solvent contains an additional surfactant. The dissolution rate increases so markedly that about 90 percent of the composition has passed into solution after a time lapse of only about two minutes, even when no surfactant whatsoever is present in the water solvent. This constitutes an important and unexpected improvement with respect to prior art techniques.

The present invention, in its various embodiments, enables the manufacture of pharmaceutical compositions which contain higher quantities of active pharmaceutical substance than was earlier considered possible in practice, while nevertheless exhibiting an equally as rapid or even more rapid release of the pharmaceutical substance when dissolved in water, and thereby a more rapid therapeutic effect. This constitutes a considerable and unexpected step forward in the field of pharmaceutical formulation.

## Claims

1. A pharmaceutical composition for rapid release of pharmaceutic substance and containing at least one finely divided, hydrophobic pharmaceutical substance which is present in a finely dispersed or dissolved state in a water-soluble carrier together with a finely dispersed or dissolved surfactant, characterized in that said substance or substances and said surfactant have been incorporated into said carrier while the carrier has been heated to a molten state, to form an intimate mixture with each other, and said mixture has then been cooled to solidify into said pharmaceutical composition.

2. A composition according to claim 1, characterized in that the surfactant is present in an amount of 0.5 - 5 weight percent, and preferably 0.5 - 3 weight percent, of the composition.

3. A composition according to claim 1 or 2, characterized in that the pharmaceutical substance has an average particle size of at most about 10 µm and preferably at most about 6 µm.

4. A composition according to any of claims 1-3, characterized in that the carrier consists of a material in which the pharmaceutical substance will dissolve at an elevated temperature but is not readily dissolved at room temperature.

5. A composition according to claim 4, characterized in that the carrier consists of a material which is liquid at the temperature at which the pharmaceutical substance is soluble, but is solid at room temperature.

6. A pharmaceutical preparation, characterized in that it contains at least one pharmaceutical composition according to any on of claims 1-5

## Patentansprüche

1. Pharmazeutische Zusammensetzung für die rasche Freisetzung von pharmazeutischen Substanzen, enthaltend mindestens eine fein verteilte, hydrophobe pharmazeutische Substanz, die in einem fein dispergierten oder gelösten Zustand in einem wasserlöslichen Träger zusammen mit einem fein dispergierten oder gelösten oberflächenaktiven Mittel vorliegt, dadurch gekennzeichnet, daß die Substanz bzw. Substanzen und das oberflächenaktive Mittel dem Träger zugesetzt wurden, während der Träger bis zu einem geschmolzenen Zustand erhitzt wurde, um ein inniges Gemisch derselben zu ergeben, und das Gemisch anschließend abgekühlt wurde, um sich unter Bildung der pharmazeutischen Zusammensetzung zu verfestigen.

2. Zusammensetzung nach Anspruch 1, dadurch gekennzeichnet, daß das oberflächenaktive Mittel in einer Menge von 0,5 - 5 Gew.-% und vorzugsweise von 0,5 - 3 Gew.-% in der Zusammensetzung vorhanden ist.

3. Zusammensetzung nach Anspruch 1 oder 2, dadurch gekennzeichnet, daß die pharmazeutische Substanz eine durchschnittliche Teilchengröße von maximal etwa 10 µm und vorzugsweise von maximal etwa 6 µm aufweist.

4. Zusammensetzung gemäß einem jeden der Ansprüche 1 bis 3, dadurch gekennzeichnet, daß der Träger aus einem Material besteht, in dem sich die pharmazeutische Substanz bei erhöhter Temperatur löst, jedoch bei Raumtemperatur nicht leicht löslich ist.

5. Zusammensetzung nach Anspruch 4, dadurch gekennzeichnet, daß der Träger aus einem Material besteht, das bei der Temperatur flüssig ist, bei der die pharmazeutische Substanz löslich ist, bei Umgebungstemperatur jedoch fest ist.

6. Pharmazeutische Zubereitung, dadurch gekennzeichnet, daß sie mindestens eine pharmazeutische Zusammensetzung gemaß einem jeden der Ansprüche 1 bis 5 enthält.

## Revendications

1. Composition pharmaceutique pour la libération rapide d'une substance pharmaceutique et contenant au moins une substance pharmaceutique hydrophobe, finement divisée, qui est présente à l'état finement dispersé ou dissous dans un véhicule hydrosoluble conjointement avec un agent tensiomotif finement dispersé ou dissous, caractérisée en ce que la ou les substances et l'agent tensioactif ont été incorporés dans ce véhicule tandis que le véhicule a été chauffé jusqu'à l'état de fusion pour former un mélange intime réciproque et ce mélange a ensuite été refroidi pour la solidification en la composition pharmaceutique.

2. Composition selon la revendication 1, caractérisée en ce que l'agent tensioactif est présent dans une quantité de 0,5 - 5 % en poids, et de préférence de 0,5 - 3 % en poids de la composition.

3. Composition selon la revendication 1 ou 2, caractérisée en ce que la substance pharmaceutique a une granulométrie moyenne d'au plus environ 10 µm et de préférence d'au plus environ 6 µm.

4. Composition selon l'une quelconque des revendications 1-3, caractérisée en ce que le véhicule consiste en un matériau dans lequel la substance pharmaceutique va se dissoudre à une température élevée, mais n'est pas facilement dissoute à température ambiante.

5. Composition selon la revendication 4, caractérisée en ce que le véhicule consiste en un matériau qui est liquide à la température à laquelle la substance pharmaceutique est soluble, mais qui est solide à température ambiante.

6. Préparation pharmaceutique, caractérisée en ce qu'elle contient au moins une composition pharmaceutique selon l'une quelconque des revendications 1-5.
